# EUROPÄISCHE PATENTSCHRIFT

(11) **EP 0 409 020 B1**
(45) Veröffentlichungstag und Bekanntmachung des Hinweises auf die Patenterteilung: **21.09.1994**
(21) Anmeldenummer: 90112996.5
(22) Anmeldetag: 07.07.1990
(51) Int. Cl.: F16K 7/06, F16H 25/12, A61M 39/00

(54) **Vorrichtung zum Klemmen von Schläuchen**
Device for squeezing tubes
Dispositif pour serrer un tuyau

(30) Priorität: 19.07.1989 DE 3923837
(43) Veröffentlichungstag der Anmeldung: 23.01.1991
(73) Patentinhaber: Fresenius AG, 61350 Bad Homburg (DE)
(72) Erfinder: Flaig, Hans-Jürgen, Dr., D-6420 Lauterbach (DE); Jahn, Paul, D-6000 Frankfurt am Main 56 (DE)
(74) Vertreter: Fuchs Mehler Weiss

(56) Entgegenhaltungen:
- EP-A- 0 319 274
- DE-A- 3 515 855
- GB-A- 2 071 222
- US-A- 3 550 619
- US-A- 4 303 222
- US-A- 4 640 144

## Beschreibung

Die Erfindung bezieht sich auf eine Vorrichtung zum Klemmen von Schläuchen.

Im Bereich der Medizintechnik, insbesondere bei extrakorporalen Blutkreisläufen besteht häufig die Notwendigkeit, Schläuche mittels Klemmen in gezielter Weise zu öffnen bzw. abzusperren. Ein typisches Anwendungsgebiet ist die Dialyse im extrakorporalen Kreislauf unter Verwendung einer Single-Needle-Anordnung. Während eines Bedienungszyklus müssen die jeweiligen Schlauchbereiche des arteriellen und des venösen Schenkels geöffnet bzw. verschlossen werden. Dies erfolgt über entsprechende Klemmen.

Die aus dem Stand der Technik bekannten Klemmvorrichtungen verwenden Hub- oder Drehmagnete, welche mit einer entsprechenden Mechanik betriebsverbunden sind. Der entscheidende Nachteil dieser Magnete besteht darin, daß diese eine sehr hohe Stromaufnahme aufweisen und im betätigten Zustand zu einer erheblichen Wärmeabgabe führen. Ein weiterer Nachteil der Magneten ist die hohe Geräuschentwicklung, welche insbesondere bei einer mehrstündigen Dialyse zu starken Beeinträchtigungen und Störungen des Patienten führen kann.

Die DE 33 44 849 A1 zeigt eine Sicherheitseinrichtung für Infusionsregelgeräte, bei welcher ein Schlauch mittels eines Schwerts klemmbar ist, welches quer zum Schlauch verschiebbar gelagert ist. Die Betätigung des Schwertes folgt über eine auf einer Abtriebswelle eines Getriebes angeordnete Nocke. Das Gerät dient als Sicherheitseinrichtung, welche insbesondere bewirken soll, daß bei einem Stromausfall der Infusionsvorgang unterbrochen wird. Daraus ergibt sich, daß das Gerät zu einem kontinuierlichen Öffnen und Schließen eines Schlauches während des Infusionsbetriebes nicht geeignet ist, da insbesondere das Getriebe selbst sowie der Stellmotor eine hohe Trägheit aufweisen und nicht soweit spielfrei auszugestalten sind, daß eine sichere und schnell ansprechende Betätigung der Ventilfunktion, welche das Schwert ausüben soll, gewährleistet ist.

Die DE 31 04 985 A1 zeigt eine Förder- oder Pumpvorrichtung, bei welcher ein Schlauch mittels zweier Klemmelemente wahlweise abklemmbar ist, während in dem Bereich zwischen den beiden Klemmelementen ein Pumpstößel angeordnet ist, mit Hilfe dessen das zwischen den beiden Klemmelementen befindliche Flüssigkeitsvolumen in den Schlauch verdrängt werden kann. Ein Antrieb erfolgt bei dieser Vorrichtung über eine Nockenvelle, welche mit Nocken versehen ist, die mit dem Pumpelement bzw. den Klemmelementen in Betriebsverbindung stehen. Diese Vorrichtung ist nicht dazu geeignet, eine exakte und betriebssichere Klemmung eines Schlauches einer Infusionseinrichtung zu bewirken, da die Klemmeinrichtungen lediglich im Hinblikck auf den Pump- oder Fördervorgang konzipiert sind.

Aus der DE-OS 15 41 363 ist eine Infusionseinrichtung vorbekannt, bei welcher ein Klemmen des Schlauches mittels eines Elektromagneten erfolgt und welche somit die bereits in der Beschreibungseinleitung angegebenen Nachteile aufweist.

Die DE-A-15 41 363 beschreibt ein Dosiergerät für Infusionseinrichtungen. Bei diesen wird zum Klemmen eines Schlauches ein längsverschiebbar an einem Träger gelagerter StöSel gegen den Schlauch gedrückt. Die zum Klemmen des Schlauches erforderliche Kraft wird mittels eines elastischen Vorspannelements (Druckfeder) aufgebracht. Bei Betätigung des Elektromagneten wird die Kraft des Vorspannelementes überwunden, so daß der Stößel zurückbewegt wird. Hierdurch wird der Schlauch freigegeben.

Die EP-A1-319 274 beschreibt ein mechanisches Antriebssystem für ein medizinisches Infusionssystem. Das Antriebssystem wird verwendet, um eine Pumpe und aktive Ventile, welche in einer Kassette vorgesehen sind, zu betätigen. Zu diesem Zwecke weist die Pumpe längsverschiebbar gelagerte Stößel auf, welche mittels eines Nockenelementes betätigbar sind. Die Stößel liegen an der Unterseite des Nockenelementes, welche als Nockenbahn ausgebildet ist, an. Das Antriebssystem ist so ausgebildet, daß mehrere Stößel durch ein Nokkenelement betätigbar sind. Es erfolgt jedoch eine Vorspannung der Stößel von den Ventilkörpern weg in Richtung auf die Nockenbahn.

Der Erfindung liegt die Aufgabe zugrunde, eine Vorrichtung der eingangs genannten Art zu schaffen, welche bei einfachem Aufbau und betriebssicherer Anwendbarkeit unter Vermeidung störender Einflüße, insbesondere auf den Patienten, während eines langen Betriebszeitraumes betätigbar ist.

Erfindungsgemäß wird die Aufgabe durch die Merkmale des Hauptanspruchs gelöst, die Unteransprüche zeigen weitere vorteilhafte Ausgestaltungen der Erfindung.

Die erfindungsgemäße Vorrichtung zeichnet sich durch eine Reihe erheblicher Vorteile aus. Die Verwendung von Stößeln gestattet es, diese an die jeweiligen Schlauchquerschnitte anzupassen und so auszubilden, daß bei Betätigung der Vorrichtung der Schlauch in zuverlässiger Weise geklemmt bzw. freigegeben wird. Da die Betätigung der Stößel erfindungsgemäß über ein Nockenelement erfolgt, welches mittels eines Antriebs bewegbar ist, können die Stößel auf rein mechanischem Wege gegen den Schlauch vorgespannt bzw. von diesem zurückgezogen werden. Die Bewegung der Stößel als solche ist dabei nicht durch elektrische Antreibe zu bewirken, es ist erfindungsgemäß lediglich nötig, das Nockenelement selbst zu bewegen. Da hierfür wesentlich geringere Kräfte als zum Klemmen des Schlauches erforderlich sind, kann die Antriebsleistung und damit die Stromaufnahme erheblich verringert werden. Erfindungsgemäß ist es insbesondere von Vorteil, daß eine Energiebeaufschlagung der Vorrichtung nur bei Veränderungen des Zustandes erforderlich sind, während in den Stillstandsphasen kein zusätzlicher Energieeintrag erforderlich ist. Die aus dem Stand der Technik bekannten Probleme hinsichtlich der Wärmeentwicklung und der Energieaufnahme entfallen erfindungsgemäß somit vollständig. Weiterhin kann ein Nockenelement geräuscharm bewegt werden, da zum Öffnen und Schließen der Vorrichtung jeweils nur geringe Verschiebewege der Stößel erforderlich sind. Ein weiterer Vorteil des erfindungsgemäßen Nockenelements besteht darin, daß dieses geräuscharm betätigt werden kann, da die bei Hubmazgneten bekannten schlagartigen Beschleunigungen bzw. Abbremsungen der Ankerelemente nicht vorkommen. Das einzige bei der erfindungsgemäßen Vorrichtung auftretende Geräusch besteht in dem Laufgeräusch des Antriebs, beispielsweise eines Elektromotors oder Schrittmotors, welcher zusätzlich mit einem Getriebe versehen sein kann. Die erfindungsgemäße Vorrichtung eignet sich somit insbesondere auch zur Langzeitanwendung bei sehr geräuschempfindlichen Patienten.

In einer bevorzugten Ausführungsform der Erfindung ist vorgesehen, daß das Nockenelement in Form einer drehbar gelagerten Nockenscheibe ausgebildet ist. Die drehbare Ausbildung der Nockenscheibe weist den Vorteil auf, daß diese besonders klein und einfach ausgestaltet sein kann.

Die erfindungsgemäße Nockenscheibe ist an zumindest einer Fläche mit einer Nockenbahn versehen, welche erhabene und zurückversetzte Bereiche aufweist. Die Niveauunterschiede zwischen den erhabenen und zurückversetzten Bereichen entsprechen der Longitudinalbewegung der Stößel, d.h. in etwa dem Schlauchdurchmesser. Erfindungsgemäß steuert somit die Nockenbahn direkt, ohne Zwischenschaltung eines weiteren Getriebes, die Bewegung der Stößel. Weiterhin ist durch die Niveauunterschiede zwischen den erhabenen und zurückversetzten Bereichen sichergestellt, daß die Stößel jeweils vollständig über die erforderlich Weglänge bewegt werden. Zwischenstellungen, welche zu Fehlfunktionen führen könnten, sind damit ausgeschlossen.

Bevorzugterweise ist die Nockenbahn kreisringförmig ausgebildet. Dies führt zu dem Vorteil, daß die Herstellung der Nockenbahnen sehr einfach und kostengünstig erfolgen kann.

Um eine sichere Führung des jeweiligen Stößels an dem Nokkenelement sicherzustellen, ist erfindungsgemäß vorgesehen, daß der Stößel an seinem einen Endbereich mit einem entlang der Nockenbahn bewegbaren Führungselement ausgebildet ist. Bevorzugterweise ist dieses Führungselement ein Rad, welches an einer senkrecht zur Längsachse des Stößels angeordneten Achse drehbar ist und auf der Nockenbahn abrollt. Durch das Rad ergeben sich nur geringfügige Reibungskräfte, weiterhin kann ein im wesentlichen geräuschloser Lauf des Führungselementes sichergestellt werden.

Um eine exakte Bewegung der Nockenbahn sicherzustellen, ist der Antrieb bevorzugterweise in Form eines Schrittmotors ausgebildet, wobei es hierbei möglich ist, die kreisringförmige Nockenbahn beispielsweise umlaufend zu bewegen oder reversierend Hin- und Herzudrehen.

Da bei der erfindungsgemäßen Vorrichtung eine zwangsweise Bewegung des Stößels in beiden Bewegungsrichtungen erforderlich ist, ergeben sich verschieden Ausgestaltungsmöglichkeiten. So ist es beispielsweise möglich, den Stößel in einer Bewegungsrichtung mittels eines elastischen Vorspannelements vorzuspannen, während die Bewegung in der anderen Bewegungsrichtung über das Nockenelement bewirkt wird.

Erfindungsgemäß bestimmt die Zwangsführung der jeweiligen Stößel deren Bewegung, so daß zusätzliche Kontrollmaßnahmen grundsätzlich nicht erforderlich wären. Es ist jedoch erfindungsgemäß für verschiedene Anwendungszwecke günstig, zusätzlich an dem Träger ein Sensorelement anzuordnen, mit Hilfe dessen die Stellung des Stößels oder in einer anderen Version die Stellung der Nockenbahn bestimmt werden kann. Das Sensorelement kann beispielsweise in Form einer Lichtschranke ausgestaltet sein.

Die erfindungsgemäße gestattet die Anbringung mehrerer Stößel, so daß mittels einer einzigen erfindungsgemäßen Vorrichtung eine Vielzahl von Schläuchen eines extrakorporalen Kreislaufs geklemmt oder freigegeben werden können. Dabei erweist es sich als besonders vorteilhaft, daß nur ein einziger Stellmotor erforderlich ist, mit Hilfe dessen ein Nockenelement betätigt wird, welches zur gleichzeitigen Verschiebung mehrerer Stößel dienen kann. Die Stößel können sich mit verschiedenen Bereichen der Nockenbahn in Eingriff befinden, es ist auch möglich, mehrere Stößel jeweils mit demselben Bereich der Nockenbahn in Eingriff zu halten, wobei im letzteren Falle die Stößel nicht mehr individuell voneinander betätigbar sind, sondern jeweils die zugeordneten Stößel gleichzeitig auf- oder abbewegt werden.

In einer Weiterbildung der Erfindung ist es auch möglich, durch jeden Stößel einen Schlauch zu klemmen oder die Stößel so auszubilden, daß durch diese mehrere Schläuche, welche beispielsweise nebeneinanderliegend vorgesehen sind, geklemmt oder freigegeben werden können. Weiterhin ermöglicht es die Vorrichtung, mehrere Nockenelemente und Stößel mittels nur eines Antriebs zu betätigen.

Im folgenden wird die Erfindung anhand von Ausführungsbeispielen in Verbindung mit der Zeichnung beschrieben. Dabei zeigt:
- Fig. 1: eine schematische Seitenansicht eines ersten Ausführungsbeispiels der erfindungsgemäßen Vorrichtung,
- Fig. 2: eine schematische Teilansicht einer abgewandelten Ausgestaltungsform der in Fig. 1 gezeigten Vorrichtung,
- Fig. 3: eine Draufsicht auf ein erfindungsgemäßes Nockenelement
- Fig. 4: eine Draufsicht auf ein weiteres erfindungsgemäßes Nockenelement,
- Fig. 5: eine weitere Seitenansicht, ähnlich Fig. 1, eines Ausführungsbeispieles der erfindungsgemäßen Vorrichtung,
- Fig. 6: eine Draufsicht auf das in Fig. 5 verwendete Ausführungsbeispiel der Nockenscheibe,
- Fig. 7: eine Seitenansicht der in Fig. 6 gezeigten Nockenscheibe auf die Linke Seite gemäß Fig. 6,
- Fig. 8: eine Seitenansicht des in Fig. 6 dargestellten Ausführungsbeispieles der Nockenscheibe auf die obere Seite gemäß Fig. 6 und
- Fig. 9: eine Abwicklung der Nockenbahn der in den Fig. 6 bis 8 gezeigten Nockenscheibe.

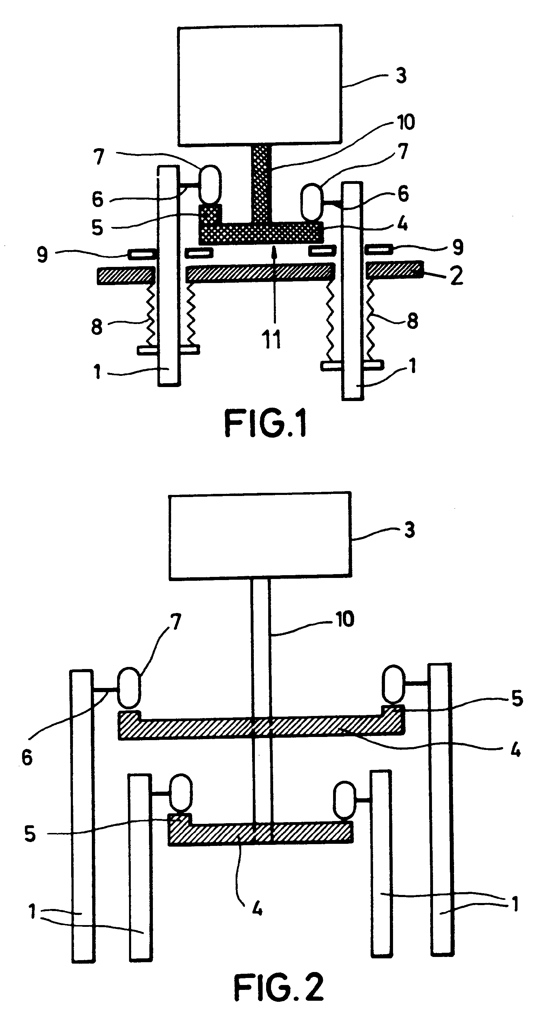

Die erfindungsgemäße Vorrichtung umfaßt einen Träger 2, welcher in Fig. 1 nur schematisch dargestellt ist. An dem Träger 2 sind mehrere Stößel 1 gelagert, welche stab- oder stangenförmig ausgebildet sind und an ihrem unteren Endbereich jeweils gegen einen nicht gezeigten Schlauch spannbar sind. Die Fig. 1 zeigt aus Gründen der Vereinfachung nicht die Ausbildung der unteren Enden der Stößel, welche den Schläuchen speziell angepaßt sein kann.

Die verschiebbar an dem Träger 2 gelagerten Stößel 1 weisen an ihrem oberen Endbereich eine quer zur Längsrichtung des Stößels 1 angeordnete Achse 6 auf, an welcher eine Führungselement 7, welches in Form eines Rades ausgebildet ist, drehbar gelagert ist.

Weiterhin sind an dem Träger 2 jeweils Sensorelemente 9 vorgesehen, welche beispielsweise in Form einer Lichtschranke o.ä. ausgebildet sein können, um die jeweilige Position des Stößels 1 festzustellen. Außerdem ist an dem Träger 2 ein Sensorelement 11 vorgesehen, welches beispielsweise in Form einer Reflexionslichtschranke ausgebildet sein kann, um die Position der Nockenscheibe 4 festzustellen.

Oberhalb des Trägers 2 ist ein Antrieb (Schrittmotor) 3 vorgesehen, dessen Achse 10 an ihrem unteren Ende eine Nockenscheibe 4 trägt, welche mit einer kreisringförmigen Nockenbahn 5 versehen ist. Auf der Nockenbahn 5 laufen die Räder 7 der Stößel 1, so daß eine Drehung der Nockenscheibe 4 zu einer Auf- bzw. Abbewegung der Stößel führt.

Da bei dem gezeigten Ausführungsbeispiel die Stößel durch die Nockenbahn 5 nur in einer nach oben gerichteten Bewegungsbahn geführt werden, ist unterhalb des Trägers 2 jeweils ein Vorspannelement 8 in Form einer Druckfeder vorgesehen. Dieses spannt den Stößel 1 in eine Klemmstellung vor, so wie dies auf der rechten Bildhälfte von Fig. 1 dargestellt ist. Die linke Bildhälfte der Fig. 1 zeigt den Stößel 1 im zurückgezogenen Zustand.

Das in Fig. 2 gezeigte Ausführungsbeispiel unterscheidet sich von dem Ausführungsbeispiel gemäß Fig. 1 dadurch, daß an der Achse 10 zwei Nockenscheiben 4 vorgesehen sind, welche jeweils eine Nockenbahn 5 aufweisen und zur Betätigung jeweils zweier Stößel 1 dienen.

Es ergibt sich für den Fachmann, daß erfindungsgemäß nicht lediglich zwei Stößel vorhanden sein müssen, es ist auch möglich, nur einen Stößel oder eine Vielzahl von Stößeln zu verwenden.

Eine Überwachung der jeweiligen Stellung der Stößel kann entweder in Abhängigkeit von der Drehstellung der Nockenscheibe 4 erfolgen, oder durch Überwachung durch das Sensorelement 9. Die Überwachung durch das Sensorelement bringt den Vorteil, daß bei einer Betriebsstörung, beispielsweise einem Bruch des Vorspannelements 8 der Fehler sofort erkannt werden kann.

Bei Verwendung eines Schrittmotors erweist es sich als günstig, daß dieser einen sehr geringen Haltestrom erforderlich macht, welcher üblicherweise nur rd. 20 % des Betriebsstroms ausmacht.

Die erfindungsgemäße Vorrichtung gestattet es, die jeweiligen Stößel 1 individuell und unabhängig voneinander zu bewegen. Zu diesem Zwecke weist die kreisscheibenförmige Nokkenscheibe 4, wie bereits erwähnt, eine kreisringartige Nockenbahn 5 auf, deren Erhebungen und zurückversetzte Bereiche jeweils über geeignete Schrägen ineinander übergehen, um ein Abrollen des Führungselements 7 zu erleichtern.

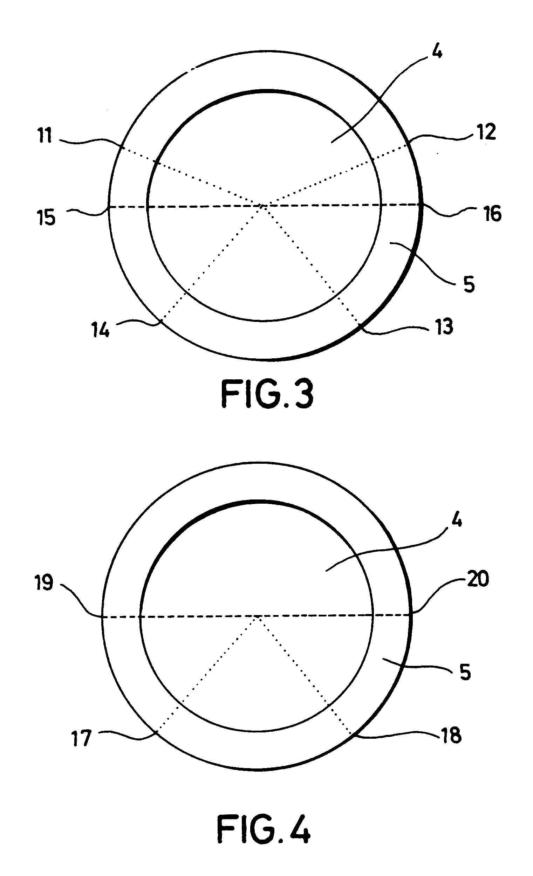

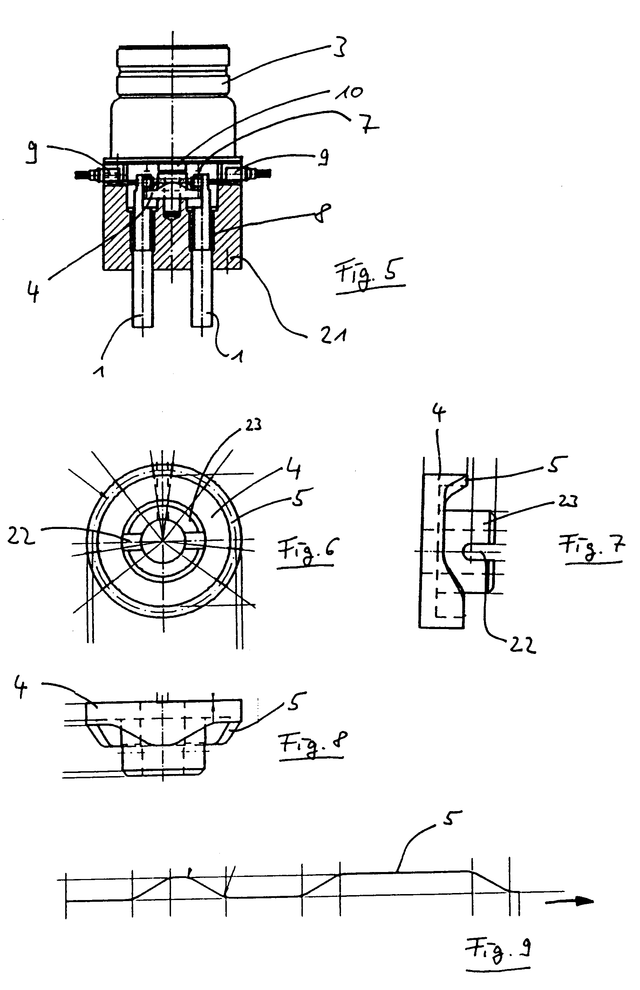

Die Fig. 3 und 4 zeigen jeweils Draufsichten auf unterschiedliche Ausführungsbeispiele einer erfindungsgemäßen Nockenscheibe. Das in Fig. 3 gezeigte Ausführungsbeispiel ist für zwei Stößel 1 vorgesehen. Die Nockenbahn 5 weist hierbei erhabene bzw. abgesenkte Bereiche auf, so wie dies in Fig. 1 schematisch dargestellt ist. Bei dem Ausführungsbeispiel liegt eine Erhebung der Nockenbahn zwischen den Punkten 11 und 12 vor, eine weitere, kürzere Erhebung ist zwischen den Punkten 13 und 14 vorgesehen. Die Höhe der Erhebung der Nockenbahn 5 ergibt sich aus dem maximal möglichen und erwünschten Hub des Stößels 1. In einer vorteilhaften Ausführung ist der längere erhabene Bereich zwischen den Punkten 11 und 12 in einem Winkelbereich von 105° vorgesehen, während der kürzere Bereich sind über einen Winkel von 50° erstreckt. Der Anstieg an der rechten und linken Seite, welcher zur Erreichung des jeweiligen erhabenen Bereichs erforderlich ist, überstreicht einen Winkel von 30°. Bei dem gezeigten Ausführungsbeispiel ist der Niveauunterschied zwischen dem erhabenen und dem abgesenkten Bereich 3 mm, so daß der Stößel insgesamt um 3 mm gehoben bzw. gesenkt werden kann. In der O-Stellung der Nockenscheibe stehen die jeweiligen Räder 7 an den Punkten 15 und 16. Diese stellen abgesenkte Bereiche dar, in welchen die jeweiligen Schläuche geklemmt sind.

Zu Beginn des Betriebes wird die O-Stellung der Nockenscheibe angefahren. Durch Drehen der Nockenscheibe um 45° im Uhrzeigersinn wird der in Fig. 1 rechts gezeigte Stößel zurückgezogen. Durch ein weiteres Drehen um 45° in derselben Richtung wird auch der in Fig. 1 links gezeigte Stößel zurückgezogen. Nach Zurückdrehen um denselben Winkel fährt der linke Stößel wieder aus, ein weiteres Zurückdrehen erlaubt dem rechten Stößel eine Ausfahrbewegung. Bedingt durch die Symmetrie der Nocken ist eine Bewegung der Stößel in umgekehrter Reihenfolge durch eine Umkehr der Drehrichtung aus der O-Lage möglich.

Die Fig. 4 zeigt ein weiteres Ausführungsbeispiel, bei welchem zwischen den Punkten 17 und 18 eine Erhöhung der Nokkenbahn vorgesehen ist. Die Erhöhung besteht aus einer Rampe mit einem Winkel von 30° und einem Plateau, welches sich über einen Winkel von 15° erstreckt. Zu Beginn des Betriebs wird die O-Stellung angefahren (Punkte 19 und 20). Eine Drehung der Nockenscheibe im Uhrzeigersinn um 90° führt zu eiher Anhebung des in Fig. 1 links gezeigten Stößels, während bei einer Drehung im Gegenuhrzeigersinn um den gleichen Winkelbetrag der in Fig. 1 rechts gezeigte Stößel aus der O-Stellung angehoben wird. Bei dieser Ausführungsform ist jederzeit gewährleistet, daß zumindest eine der beiden Klemmen geschlossen ist.

Die erfindungsgemäße Vorrichtung ermöglicht es, die einzelnen Stößel separat und unabhängig voneinander zu betätigen, die Bewegung der Stößel jedoch so zuzuordnen, daß ein entsprechender Funktionsablauf der jeweiligen Anlage sichergestellt ist. Da jeweils mehrere Stößel durch eine Nockenbahn betätigt werden, ist zugleich sichergestellt, daß die Bewegung der beiden Stößel zueinander angepaßt ist, ein Ausfall nur eines Stößels, wie dies bei Hubmagneten der Fall sein kann, ist nicht möglich. Insofern ergibt sich eine wesentliche Steigerung der Betriebssicherheit.

Durch geeignete Anordnungen der Stößel 1 am Umfang der Nokkenscheibe 4 ist es möglich, die verschiedensten Bewegungsabfolgen der Stößel zu realisieren. Zusätzlich zu der geringeren Stromaufnahme und geringeren Wärmeentwicklung der erfindungsgemäßen Vorrichtung ist ein kompakter Aufbau möglich. Weiterhin kann die Vorrichtung besonders leise betrieben werden. Die mechanische Kopplung der Stößel erhöht in beträchtlichem Maße die Betriebssicherheit.

Die Fig. 5 zeigt eine Seitenansicht, teils im Schnitt, eines weiteren Ausführungsbeispieles der erfindungsgemäßen Vorrichtung. Gleiche Teile wurden mit gleichen Bezugszeichen versehen. Aus der Abbildung von Fig. 5 ergibt sich, daß die Stößel 1 in Bohrungen eines Gehäuses 21 geführt sind, wobei die Stößel 1 abgestuft ausgebildet sind und an ihrem oberen, mit einem geringeren Durchmesser versehenen Bereich von der Spiralfeder 8 (Vorspannelement) umgeben sind. An der Oberseite der Gehäuses 21 ist der Antriebsmotor 3 angeflanscht. Die Sensoreinrichtungen sind seitlich in das Gehäuse 21 eingesetzt.

Die Fig. 6 bis 8 zeigen in der Draufsicht sowie in zwei Seitenansichten jeweils ein Ausführungsbeispiel der mit einer Nockenbahn 5 versehen Nockenscheibe 4. Bei diesem Ausführungsbeispiel ist zu erkennen, daß die Nockenscheibe 4 mit einer Nabe 23 versehen ist, welche von einer Nut 22 quer durchkreuzt wird. Die Nut 22 ermöglicht es, daß die beiden Sensoren 9 (siehe Fig. 5) bei einer bestimmten Stellung der Nockenscheibe 4 ein optisches Signal (Sender und Empfänger) übermitteln können, um auf diese Weise die Drehstellung der Nockenscheibe (4) ermitteln zu können.

Aus der Darstellung von Fig. 9 ergibt sich eine Abwicklung der in den Fig. 6 - 8 dargestellten Nockenbahn, wobei der nach rechts gerichtete Pfeil die Rollenablauflinie oder -richtung markiert.

Die Erfindung ist nicht auf die gezeigten Ausführungsbeispiele beschränkt, es ergeben sich für den Fachmann vielmehr im Rahmen der Erfindung vielfältige Abwandlungs- und Modifikationsmöglichkeiten.

## Patentansprüche

1. Vorrichtung zum Klemmen von Schläuchen, mit
- mehreren längsverschiebbar an einem Träger (2) gelagerten Stößeln (1),
- jeweils zumindest einem elastischen Vorspannelement (8) zur Vorspannung des Stößels (1) in Klemmrichtung,
- einem mittels eines Antriebs (3) bewegbaren, eine kreisförmige Nockenbahn (5) aufweisenden Nockenelement (4) zur Betätigung der Stößel (1), wobei die Nockenbahn (5) erhabene und rückversetzte Bereiche aufweist,
- wobei das Nockenelement (4) als Scheibe ausgebildet ist, die um eine parallel zur Längsachse der Stößel (1) angeordnete Achse drehbar ist,
- einem am Endbereich jedes Stößels (1) gelagerten, längs der Nockenbahn (5) bewegbaren Führungselement (7),
- wobei die Führungselemente (7) auf der Nockenbahn (5) mittels der Vorspannkraft aufliegen.

2. Vorrichtung nach Anspruch 1, dadurch gekennzeichnet, daß der Stößel (1) an seinem Endbereich eine senkrecht zu seiner Längsachse gelagerte Achse (6) aufweist, auf welcher drehbar ein das Führungselement (7) bildendes Rad gelagert ist.

3. Vorrichtung nach einem der Ansprüche 1 oder 2, dadurch gekennzeichnet, daß an dem Träger (2) ein Sensorelement (9) zur Bestimmung der Stellung des Stößels (1) angeordnet ist.

4. Vorrichtung nach einem der Ansprüche 1 bis 3, dadurch gekennzeichnet, daß an dem Träger (2) ein weiteres Sensorelement (11) zur Bestimmung der Stellung der Kurvenscheibe (4) angeordnet ist.

5. Vorrichtung nach einem der Ansprüche 1 bis 4, dadurch gekennzeichnet, daß mehrere Nockenelemente (4) und Stößel (1) mittels eines Antriebs (3) betätigbar sind.

## Claims

1. An apparatus for clamping for clamping flexible tubes, comprising
- several plunger means (1) being longitudinally displaceable along a support means (2),
- at least one elastic biasing element (8) for biasing said plunger means (1) in clamping direction,
- one cam element (4) movable by means of a drive means (3) and having an annular cam path (5) for actuating said plunger means (1) with said cam path comprising elevated and recessed portions,
- with said cam element (4) being embodied as disc rotatable about an axis which is parallel to the longitudinal axis of said plunger means (1),
- one guide element (7) mounted at the end portion of each plunger means (1) and being movable along said cam path (5),
- with said guide elements (7) being supported on said cam path (5) by means of biasing force.

2. An apparatus according to claim 1, characterized in that said plunger means (1) is provided on its end portion with an axis (6) being vertical to its longitudinal axis and on which a wheel is rotatably mounted which forms said guide element (7).

3. An apparatus according to any of claims 1 or 2, characterized in that a sensor element (9) is arranged on said support (2) for determining the position of said plunger means (1).

4. An apparatus according to any of claims 1 to 3, characterized in that a second sensor element (11) is arranged on said support (2) for determining the position of said cam disc (4).

5. An apparatus according to any of claims 1 to 4, characterized in that a plurality of cam elements (4) and plunger means (1) are actuable by means of one drive means (3).

## Revendications

1. Dispositif de serrage de tuyaux comportant
- plusieurs coulisseaux (1) montés de manière à coulisser longitudinalement sur un support (2),
- au moins un élément de précontrainte élastique (8) pour la précontrainte de chaque coulisseau (1) dans le sens du serrage,
- un élément à cames (4) déplaçable au moyen d'un mécanisme d'entraînement (3), présentant une trajectoire à cames (5) circulaire, pour l'actionnement des coulisseaux (1), la trajectoire à cames (5) présentant des zones en relief et des zones en retrait,
- l'élément à cames (4) étant un disque qui peut tourner autour d'un axe parallèle à l'axe longitudinal des coulisseaux (1),
- un élément de guidage (7) monté dans la zone d'extrémité de chaque coulisseau (1), déplaçable le long de la trajectoire à cames (5),
- les éléments de guidage (7) reposant sur la trajectoire à cames (5) sous l'effet de la force de précontrainte.

2. Dispositif selon la revendication 1, caractérisé en ce que le coulisseau (1) présente, dans sa zone d'extrémité, un axe (6) monté perpendiculairement à son axe longitudinal, sur lequel est monté tournante une roue formant l'élément de guidage (7).

3. Dispositif selon l'une des revendications 1 ou 2, caractérisé en ce que sur le support (2) est prévu un élément détecteur (9) destiné à déterminer la position du coulisseau (1).

4. Dispositif selon l'une des revendications 1 à 3, caractérisé en ce que sur le support (2) il est prévu un autre élément détecteur (11) destiné à déterminer la position du disque à cames (4).

5. Dispositif selon l'une des revendications 1 à 4, caractérisé en ce que plusieurs éléments à cames (4) et coulisseaux (1) peuvent être actionnés au moyen d'un mécanisme d'entraînement (3).
